(19) Europäisches Patentamt
European Patent Office
Office européen des brevets

(11) **EP 3 928 709 A1**

(12) **EUROPEAN PATENT APPLICATION**

(43) Date of publication:
**29.12.2021 Bulletin 2021/52**

(51) Int Cl.:
***A61B 8/00*** (2006.01)  ***A61B 8/08*** (2006.01)

(21) Application number: **20181679.0**

(22) Date of filing: **23.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PALANISAMY, Krishnamoorthy**
  **5656 AE Eindhoven (NL)**

• **PANDYA, Maulik Yogeshbhai**
  **5656 AE Eindhoven (NL)**
• **SHULEPOV, Sergei Y.**
  **5656 AE Eindhoven (NL)**
• **VAN ROOIJ, Johannes Antonius**
  **5656 AE Eindhoven (NL)**
• **JOCHEMSEN, Robert**
  **5656 AE Eindhoven (NL)**
• **DE WILD, Nico Maris Adriaan**
  **5656 AE Eindhoven (NL)**

(74) Representative: **Philips Intellectual Property & Standards**
**High Tech Campus 52**
**5656 AG Eindhoven (NL)**

(54) **SYSTEMS AND METHODS FOR IDENTIFYING A VESSEL FROM ULTRASOUND DATA**

(57) The invention provides a computer-implemented method for identifying the type of a vessel of a subject based on Doppler ultrasound data acquired from a subject. The computer-implemented method includes receiving ultrasound data acquired from the subject by way of an ultrasound probe and generating color Doppler ultrasound data from the received ultrasound data. A representation of a vessel of the subject is segmented from the color Doppler ultrasound data and pulse wave Doppler ultrasound data is obtained from the segmented vessel representation. A feature extraction algorithm is then applied to the pulse wave Doppler ultrasound data, thereby extracting a feature of the flow within the segmented vessel and the type of the vessel is identified as an artery or a vein based on the extracted feature of the flow.

Receive ultrasound data — 110

Generate color Doppler data — 120

Segment representation of a vessel — 130

Obtain pulse wave Doppler ultrasound data — 140

Apply a feature extraction data — 150

Identify vessel as artery or vein — 160

100

FIG. 2

EP 3 928 709 A1

**Description**

FIELD OF THE INVENTION

The invention relates to the field of ultrasound imaging, and more specifically to the field of vascular ultrasound imaging.

BACKGROUND OF THE INVENTION

Continuous and accurate blood flow measurements from a blood vessel are essential in hemodynamic monitoring (HDM) applications. It is possible to provide a subject with an ultrasound-based patch that will continuously quantify and monitor blood flow parameters. The subject may, for example, be in emergency and/or peri-operative care.

Full ultrasound imaging and diagnostics of the blood vessels involves a combination of structural imaging, such as B-mode imaging, and Doppler imaging, such as color flow and/or spectral Doppler imaging. In ultrasound diagnostic imaging, Doppler measurements are typically performed by skilled sonographers, wherein the sonographer positions a Doppler imaging plane in the middle of the vessel and aligned with the main axis, which extends along the length of the vessel.

However, locating such a plane in a vessel by an untrained operator can be cumbersome. Moreover, in OR/ICU environments, live image feedback on a monitor is very often not available. Further, in some examples of continuous, non-invasive blood flow monitoring using ultrasound, in particular in peri-operative care where non-ultrasound personnel are involved, traditional ultrasound scanners and image feedback are not meaningful.

In such an application, a wearable ultrasound sensor or ultrasound patch device for continuous subject monitoring may be deployed. In this case, an operator such as a nurse may be minimally involved, for example by simply placing the sensor and allowing the system to record all of the ultrasound data continuously. However, this requires the operator to place the patch correctly without seeing the actual ultrasound image of the vessel in question. When imaging vessels such as the common carotid artery (CCA), there is a high probability that the probe may be positioned over a vein rather than an artery as the internal jugular vein appears in parallel with the CCA. Accordingly, placing the patch and maintaining the positon of the patch correctly aligned with the long axis of an artery during monitoring can be extremely challenging, particularly for an unskilled sonographer.

There is therefore a need for a means of accurately and reliably identifying the vessel being measured, for example for guiding the user when positioning an ultrasound probe or patch, in a manner that is not dependent on the skill of the user.

SUMMARY OF THE INVENTION

The invention is defined by the claims.

According to examples in accordance with an aspect of the invention, there is provided a computer-implemented method for identifying the type of a vessel of a subject based on Doppler ultrasound data acquired from the subject, the method comprising:

receiving ultrasound data acquired from the subject;
generating color Doppler ultrasound data from the received ultrasound data;
segmenting a representation of a vessel of the subject from the color Doppler ultrasound data;
obtaining pulse wave Doppler ultrasound data from the segmented vessel representation;
applying a feature extraction algorithm to the pulse wave Doppler ultrasound data, thereby extracting a feature of the flow within the segmented vessel; and
identifying the type of the vessel as an artery or a vein based on the extracted feature of the flow.

The method provides for a means of identifying a vessel as either a vein or an artery based only on Doppler ultrasound data and, as the features of the flow within the vessel are not dependent on the orientation of the ultrasound probe when the ultrasound data is obtained, independent of the orientation of the ultrasound probe used to acquire the ultrasound data.

In this way, the method provides a means of performing automatic vessel identification without requiring any skilled input by a user.

In an embodiment, obtaining the pulse wave Doppler ultrasound data from the segmented vessel comprises:

identifying the center of the vessel based on the color Doppler ultrasound data;
generating a sampling volume at the center of the vessel; and

acquiring the pulse wave Doppler ultrasound data from the sampling volume.

In this way, the acquisition location of the pulse wave Doppler ultrasound data is positioned in the most accurate measurement location of the vessel, thereby increasing the accuracy of the pulse wave Doppler ultrasound data and the final vessel identification.

In an embodiment, the feature extraction algorithm comprises:

extracting a maximum frequency envelope from the pulse wave Doppler ultrasound data;
normalizing the extracted maximum frequency envelope;
determining a feature of the normalized maximum frequency envelope; and
extracting the feature of the normalized maximum frequency envelope as a feature of the flow.

Typically, the variation in the flow of an artery is higher than that of a vein and the systolic phase profile is sharper in an artery compared to a vein. By analyzing the frequency envelope of the pulse wave Doppler ultrasound data, the characteristics of the flow may be analyzed in a simple and efficient manner.

In an embodiment, determining a feature of the normalized maximum frequency envelope comprises generating a distribution of the normalized maximum frequency envelope with a histogram comprising a plurality of bins.

In an embodiment, the feature of the normalized maximum frequency envelope comprises one or more of:

a skewness of the envelope;
a maximum bin ratio; and
a bin edge roll-off

By analyzing these features, it is possible to differentiate the variations in the flow characteristic of arteries and veins.

In an embodiment, identifying the type of the vessel as an artery or a vein based on the extracted feature of the flow comprises applying a machine learning algorithm to the extracted feature.

By using a machine learning algorithm, such as one of the variety of known machine learning algorithms, the accuracy of the identification of the vessel may be improved. Further, the identification of the vessel may be performed with reduced input data.

In an embodiment, identifying the type of the vessel as an artery or a vein based on the extracted feature of the flow comprises performing a color distribution analysis on the color Doppler ultrasound data.

In this way, flow direction may also be taken into account when identifying the vessel, thereby increasing the accuracy of the identification.

In an embodiment, performing a color distribution analysis on the color Doppler ultrasound data comprises:

obtaining probe orientation data relating to the orientation of the ultrasound probe during the acquisition of the color Doppler ultrasound data; and
classifying the vessel as an artery or a vein based on a combination of probe orientation data and color Doppler data.

In this way, probe orientation may also be taken into account when identifying the vessel, thereby increasing the accuracy of the identification.

In an embodiment, the method further comprises:

generating a guidance instruction based on the identification of the vessel; and
presenting the guidance instruction to the user.

In this way, the user may be guided as to how to improve the positioning of the ultrasound probe based on the identification of the vessel.

According to examples in accordance with an aspect of the invention, there is provided a computer program comprising computer program code means which is adapted, when said computer program is run on a computer, to implement the methods described above.

According to examples in accordance with an aspect of the invention, there is provided a processor for identifying the type of a vessel of a subject based on Doppler ultrasound data acquired from a subject, wherein the processor is adapted to:

obtain ultrasound data from the subject by way of an ultrasound probe;
generate color Doppler ultrasound data from the received ultrasound data;

segment a representation of a vessel of the subject from the color Doppler ultrasound data;
obtain pulse wave Doppler ultrasound data from the segmented vessel representation;
apply a feature extraction algorithm to the pulse wave Doppler ultrasound data, thereby extracting a feature of the flow within the segmented vessel; and
identify the type of the vessel as an artery or a vein based on the extracted feature of the flow.

In an embodiment, when obtaining the pulse wave Doppler ultrasound data from the segmented vessel, the processor is adapted to:

identify the center of the vessel based on the color Doppler ultrasound data;
generate a sampling volume at the center of the vessel; and
acquire the pulse wave Doppler ultrasound data from the sampling volume.

In an embodiment, when applying the feature extraction algorithm, the processor is adapted to:

extract a maximum frequency envelope from the pulse wave Doppler ultrasound data;
normalize the extracted maximum frequency envelope;
determine a feature of the normalized maximum frequency envelope; and
extract the feature of the normalized maximum frequency envelope as a feature of the flow.

According to examples in accordance with an aspect of the invention, there is provided an ultrasound imaging system comprising:

a processor as described above; and
an ultrasound imaging probe, wherein the ultrasound probe comprises an ultrasound patch.

In an embodiment: the system further comprises a display unit, and wherein the processor is further adapted to generate a guidance instruction based on the identification of the type of the vessel and generate a display instruction to cause the display unit to display the guidance instruction; or the ultrasound patch further comprises a guidance indicator, and wherein the processor is further adapted to generate a guidance instruction based on the identification of the type of the vessel and control the guidance indicator to provide the guidance instruction to the user.
These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows an ultrasound diagnostic imaging system to explain the general operation;
Figure 2 shows a method of the invention;
Figure 3 an example of a feature extraction algorithm;
Figure 4 shows a schematic representation of the relationship between the Doppler angle, between the vessel axis and the imaging plane, and the shape of a detected ellipse;
Figure 5 shows a Doppler gate positioned at a centroid of an identified elliptical vessel cross section; and
Figure 6 shows a graphical representation of an example of guided sensor placement using a T-shaped array and visual indicators disposed on the sensor.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for identifying the type of a vessel of a subject based on Doppler ultrasound data acquired from a subject. The computer-implemented method includes receiving ultrasound data acquired from the subject by way of an ultrasound probe and generating color Doppler ultrasound data from the received ultrasound data. A representation of a vessel of the subject is segmented from the color Doppler ultrasound data and pulse wave Doppler ultrasound data is obtained from the segmented vessel representation. A feature extraction algorithm is then applied to the pulse wave Doppler ultrasound data, thereby extracting a feature of the flow within the segmented vessel and the type of the vessel is identified as an artery or a vein based on the extracted feature of the flow.

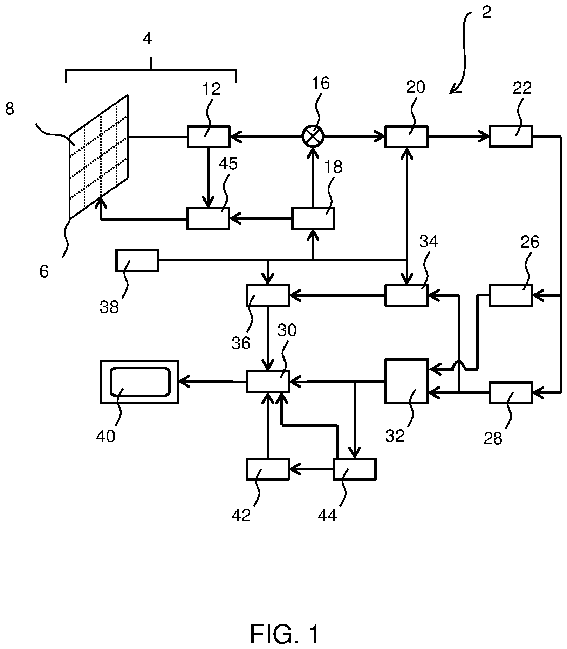

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The methods described herein may be performed on a processing unit. Such a processing unit may be located within an ultrasound system, such as the system described above with reference to Figure 1. For example, the image processor 30 described above may perform some, or all, of the method steps detailed below. Alternatively, the processing unit may be located in any suitable system, such as a monitoring system, that is adapted to receive an input relating to a subject.

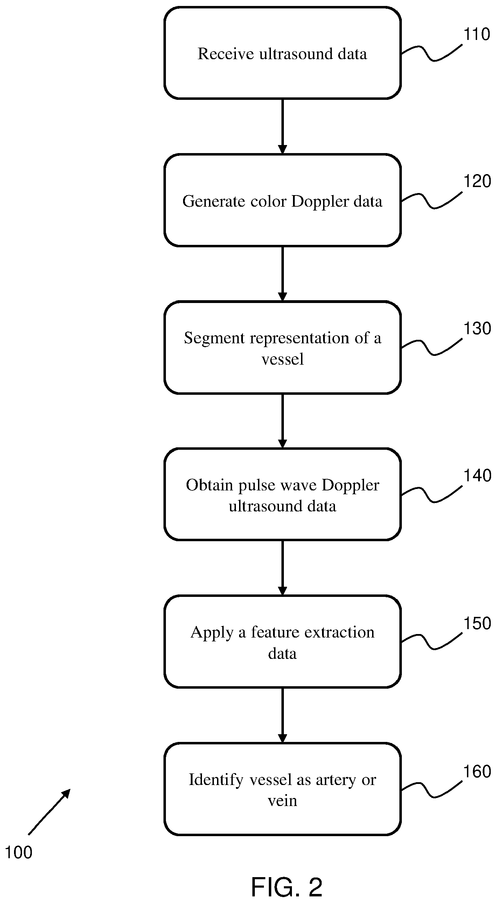

Figure 2 shows a computer-implemented method 100 for identifying the type of a vessel of a subject based on Doppler ultrasound data acquired from a subject.

The computer-implemented method begins in step 110 by receiving ultrasound data acquired from the subject by way of an ultrasound probe. Any suitable ultrasound probe may be used to obtain the ultrasound data, such as a conventional ultrasound probe or an ultrasound patch. The ultrasound probe may comprise a variety of different sensors, i.e. ultrasound transducers, such as: a linear transducer array; two linear transducer arrays, arranged such that the imaging plane of one array is orthogonal to the other; or a 2D array.

Put another way, the ultrasound probe may comprise an ultrasound patch or wearable ultrasound sensor based on an ultrasound array comprising, for example, a 1D ultrasonic arrangement or multiple 1D sub-arrays generating multi-plane datasets.

An ultrasound wearable patch may include any suitable ultrasound transducer type, such as CMUT cells or piezoelectric transducer cell. The transducer elements may be are arranged in a bi-planar configuration having a linear group of elements in the transverse orientation and another independent linear group of elements in the longitudinal orientation. In other word, the transducers may be arranged into two groups of orthogonal linear arrays. The elements arranged in the longitudinal direction may be spatially aligned with the center of the elements arranged in the transverse direction.

When acquiring the ultrasound data to be processed, either on a separate processing system at a later time or in real time on an appropriate ultrasound system, the ultrasound patch is placed in the vicinity of the CCA based on basic knowledge of the carotid anatomy, such as palpation. Upon placement of the ultrasound patch on the skin of the subject, the user may operates a button to initialize the system to begin acquiring the ultrasound data.

In step 120, color Doppler ultrasound data is generated from the received ultrasound data and in step 130 a representation of a vessel of the subject is segmented from the color Doppler ultrasound data.

In order to segment the vessel from the color Doppler ultrasound data, adapted algorithms based on a Chan-Vese-like active contour/snake concept (as described in: Chan, T.F., & Sandberg Y. B(2000). Active contours without edges for Vector-valued Image. Journal of Visual Communication and Image Representation 11, 130-141 (2000); Chan, T. F., & Vese, L. A. (2001). Active contours without edges. IEEE Transactions on Image Processing, 10(2), 266-277; and Chan, T. F., & Vese, L. A. (2002). A Multiphase level set framework for image segmentation using the Mumford and Shah model. International Journal of Computer Vision 50(3), 271-293, (2002)), and a normalized energy gradient method may be used. The latter is similar to a family of non-linear hybrid detection algorithms (such as, a combination of Sobel and Canny operators as described in Sobel, I., Feldman, G., "A 3x3 Isotropic Gradient Operator for Image Processing", presented at the Stanford Artificial Intelligence Project (SAIL) in 1968 and Canny, J., A Computational Approach To Edge Detection, IEEE Trans. Pattern Analysis and Machine Intelligence, 8(6):679-698, 1986, respectively) using optimized kernels for a given application. These methods are specifically tuned towards imaging performance of the sensor. In order to improve accuracy and robustness, a restricted region of interest (from which the data is obtained) about the principle direction may be specified and the same procedure carried out on a localized 2D area.

In step 140, pulse wave Doppler ultrasound data is obtained from the segmented vessel representation. Once the representation of the vessel has been segmented from the color Doppler ultrasound data, an accurate sampling location for obtaining pulse wave Doppler ultrasound data form the vessel may be identified.

For example, the step of obtaining the pulse wave Doppler ultrasound data from the segmented vessel may comprise identifying the center of the vessel based on the color Doppler ultrasound data, and more specifically based on the shape of the segmented representation of the vessel. The center of a vessel may be identified from the RGB patterns of the color Doppler ultrasound data to differentiate the laminar and turbulent flow within the vessel. A sampling volume may then be placed at the center of the vessel, which is the region of the vessel that possesses the maximal blood flow velocity and so is the most accurate location for measuring pulse wave Doppler ultrasound data. The pulse wave Doppler ultrasound data may then be acquired from the sampling volume.

Put another way, color Doppler ultrasound data may be provided to a processing unit where a segmentation algorithm may be used to detect the vessels using ultrasound flow information, i.e. color Doppler images, and the centers of the vessels are identified. The pulse wave Doppler ultrasound data is then acquired by placing a sample volume at the identified center of the vessels.

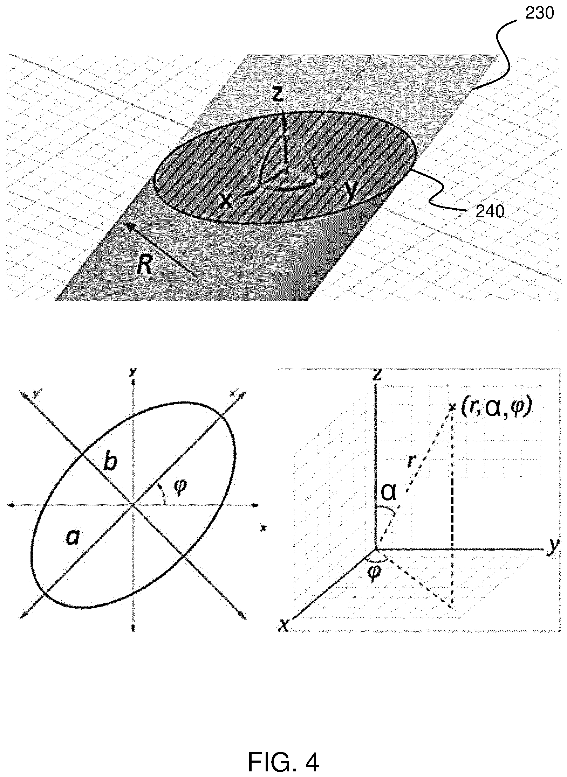

The placement of the sampling volume based at the center of the vessel is described further below with respect to Figure 4.

In step 150 a feature extraction algorithm is applied to the pulse wave Doppler ultrasound data, thereby extracting a feature of the flow within the segmented vessel and the type of the vessel is identified as an artery or a vein based on the extracted feature of the flow in step 160. Various example of feature extraction algorithms are described below.

It should be noted that the method may operate on a previously acquired dataset or a continual stream of incoming ultrasound data. Accordingly, the method may be continually repeated in order to update the identification of the currently imaged vessel based on the most recently received data. In this way, the method may compensate for subject motion or user inexperience in real time during monitoring.

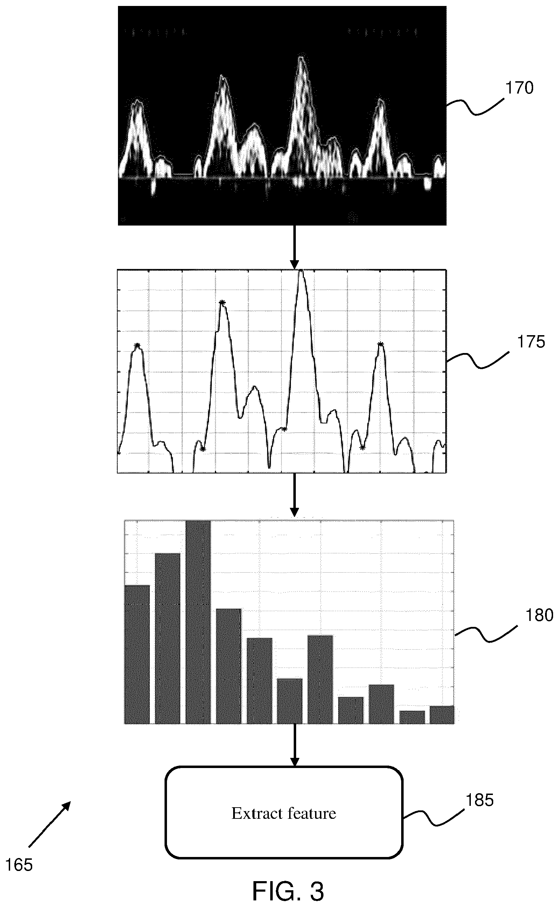

As stated above, the pulse wave Doppler ultrasound data is subjected to a feature extraction step to extract features that differentiate the artery and vein. These features are derived by considering characteristics of the flow in arteries and veins. Typically, the variations in the flow in an artery is greater than that of a vein and there exists a sharper systolic phase in an artery compared to a vein. Figure 3 shows an example 200 of a feature extraction algorithm according to an aspect of the invention.

In step 210, a maximum frequency envelope is extracted from the pulse wave Doppler ultrasound data, which is then normalized in step 220 to obtain a normalized maximum frequency envelope.

A feature of the normalized maximum frequency envelope is then identified, for example by generating a distribution of the normalized maximum frequency envelope with a histogram comprising a plurality of bins as shown in step 230.

In step 240, the feature of the normalized maximum frequency envelope, which may include one or more of: a skewness of the envelope; a maximum bin ratio; and a bin edge roll-off, is extracted as a feature of the flow.

By way of example, taking *e(n)* as the normalized envelope of the pulse wave Doppler data and *b(n)* and *c(n)* as the bin edge values and the bin counts of the histogram, respectively. The following features may be extracted according to the following equations:

Skewness of the envelope:
$$e = \frac{\frac{1}{N}\sum_{i=1}^{N}(e(n)-\bar{E})^3}{\left[\frac{1}{N-1}\sum_{i=1}^{N}(e(n)-\bar{E})^2\right]^{\frac{3}{2}}},$$
where, N is the total number samples and $\bar{E}$ is the average value of an envelope;

Maximum Bin Ratio:
$$mbr = \frac{\max[c(n)]}{\sum_{1}^{L}c(n)},$$
where, L is the total number of bins in the histogram; and

Bin edge Roll-off: *L1* such that, $\sum_{i=1}^{L1}c(n) \geq P\sum_{i=1}^{L}c(n)$ ; $0 < P < 1$ *and* $L1 < L$

These features differentiate the variations in the flow values of arteries and veins in order to identify the measured vessels.

The identification of the vessels based on the extracted features may be performed using a machine learning algorithm applied to the extracted features.

A machine-learning algorithm is any self-training algorithm that processes input data in order to produce or predict output data. Here, the input data comprises flow features and the output data comprises vessel identifications.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naïve Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example flow features. The training output data entries correspond to vessel identifications.

Put another way, a machine learning model is trained using the extracted flow features. For example, a feedforward neural network may be used with an input, two hidden layers (with four hidden neurons on each layer) and an output layer. The weights of the neurons may be obtained with a Cross-Entropy loss function and a sigmoidal activation function.

In a real-time application of the method, the same of features described above may be extracted and a similarity measure is computed with respect to the trained machine learning algorithm. The vessel which is most similar to the input feature vector is consequently labelled as either an artery or vein.

The accuracy of the classification methods described above (as computed on 50 signal samples) is tabulated below and illustrates the benefits of performing the identification based on a combination of the features described above:

| Features | Classification Accuracy |
| --- | --- |
| Skewness of the envelope | 54 |
| Maximum Bin Ratio | 58 |
| Bin edge Roll-off | 60 |
| Skewness of the envelope & Maximum Bin Ratio | 88 |
| Skewness & Bin edge Roll-off | 90 |
| Maximum Bin Ratio & Bin edge Roll-off | 88 |
| All three features | 98 |

In addition, by fixing the direction of scanning, the artery and vein can be differentiated by analyzing the color intensity values and distribution (based on RGB values) of the identified vessel segments. As the direction of the blood flow will be in opposite directions for an artery and a vein, the color distribution values may be used to differentiate an artery and vein if the probe orientation is maintained in a certain, known direction.

Put another way, identifying the type of the vessel as an artery or a vein based on the extracted feature of the flow may include performing a color distribution analysis on the color Doppler ultrasound data, for example, by obtaining probe orientation data relating to the orientation of the ultrasound probe during the acquisition of the color Doppler ultrasound data and classifying the vessel of the subject as an artery or a vein based on a combination of probe orientation data and color Doppler data. This may be used, for example, to confirm the identification of the type of the vessels derived as described above.

For example, an ultrasound wearable patch may be placed in the vicinity of the carotid with some basic prior knowledge of the carotid anatomy with a fixed probe orientation and the vessel segmented as described above. The color distribution of the identified vessels may then be analyzed individually and, based on the probe orientation, the identification of the artery and vein may be confirmed.

The shape of the vessels of the subject in the acquired color Doppler ultrasound data is typically elliptical, due to the angle of the imaging plane relative to the axis of the vessel. Figure 4 shows a schematic representation of the relationship between angle $\alpha$, the Doppler angle between the vessel axis and the imaging plane, and the shape of the detected ellipse.

Typically, when a plane crosses an infinite cylinder 230 of radius R at an angle other than 90°, the resulting shape is an ellipse 240. Taking the assumption that a blood vessel may be modeled as an infinite cylinder (i.e. there is no significant change in shape or direction of the vessel within the view of the imaging plane), the vessel cross section will be an ellipse.

When the ellipse is identified, the form-factor (*a* and *b,* dimensions of the principle axes) and its orientation ($\varphi$ and $\alpha$) derived from the color Doppler data stream are sufficient to establish the vessel axis and may be further used to vectorize the flow direction with respect to the imaging plane orientation.

The shape can be fit by a general ellipse equation, using a non-linear least-square method. In this case, the principle Euler angles and the diameter of the vessel will be directly available. Alternatively, shape recognition may be carried out directly in the imaging plane using the image momentum method.

The centroid of the shape may then be used for Doppler gate placement, for obtaining the pulse wave Doppler ultrasound data, from which the flow feature is extracted.

As the parameter relating to flow is being continually derived from a stream of Doppler ultrasound data, it is possible for the flow vector correction to adjust over time in response to external movements, such as the subject moving during examination or monitoring.

The principle angle $\varphi$ is determined by the artery orientation under the skin, while angle $\alpha$ is mainly set by the imaging plane crossing the artery.

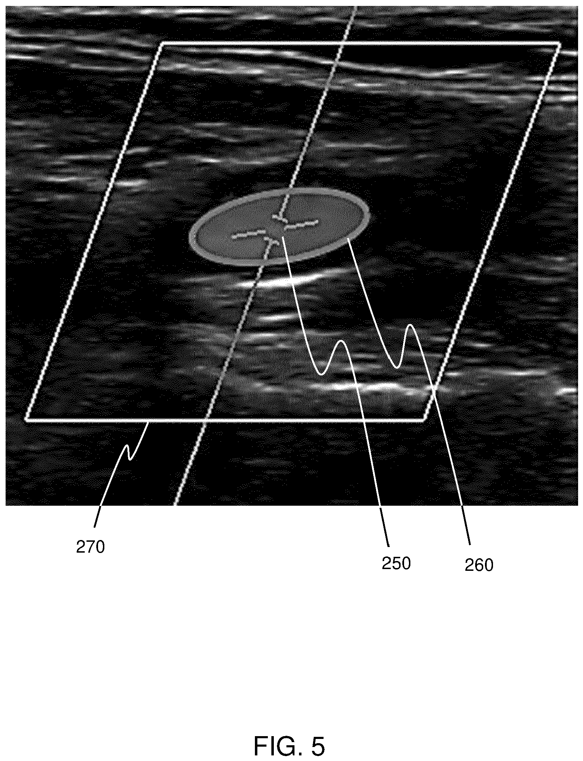

Figure 5 shows a Doppler gate, such as the sampling volume, 250 positioned at a centroid of an identified elliptical vessel cross section 260 within an imaging plane 270.

A dynamic Doppler gating concept may be employed in the algorithm. This may be realized in a number of ways. For example, a constant gating size may be used in combination with dynamic positioning of the gate over the centroid of the ellipse identified in the color Doppler data.

Alternatively, a dynamic gating size may be used, which follows the ellipsoidal shape of the color Doppler data. It should be noted that this may include a rectangular shape inscribed into an ellipse, wherein the rectangle has the same aspect ratio as said ellipse. In this case, the system selects the pulsed-wave mode gate, or sample volume size, dynamically to cover the entire vessel under evaluation.

In a further example, multiple gate positions may be used to follow the color Doppler shape, and more specifically the centroid, motion during the cardiac cycle.

The use of dynamic gating (position and/or size) may allow for: a lower mechanical index/thermal index (MI/TI) during continuous monitoring; a better rejection of artifacts during patient movement; and a more accurate reporting of the wave-form.

As discussed above, the ultrasound data is acquired by way of a sensor. There are a number of implementations of a sensor that may be used to collect the ultrasound data as described. For example, the sensor may comprise: a linear transducer array; two linear transducer arrays, arranged such that the imaging plane of one array is orthogonal to the other; or a 2D array.

Further, the user may be guided as to the placement of the sensor. The guidance provided to the user may be provided in an indirect manner. In other words, the system may analyze the ultrasound data and generate a guidance signal by way of a guidance means separate from the sensor. For example, where a visual guidance signal is generated, an arrow may be displayed on a screen indicating a direction in which the user should move the sensor.

Alternatively, the means for providing guidance to the user may be included in the sensor itself. For example, the sensor may be adapted to generate one or more of: an audible instruction; a visual instruction; an electronic control signal; and a tactile instruction, to guide the user.

The guidance may be generated based on the identification of the type of the vessel undergoing investigation and a desired vessel. For example, in the case of measuring the CCA, the user may be presented with a guidance instruction to move the probe if the vessel is identified as a vein or maintained in position if the vessel is identified as an artery.

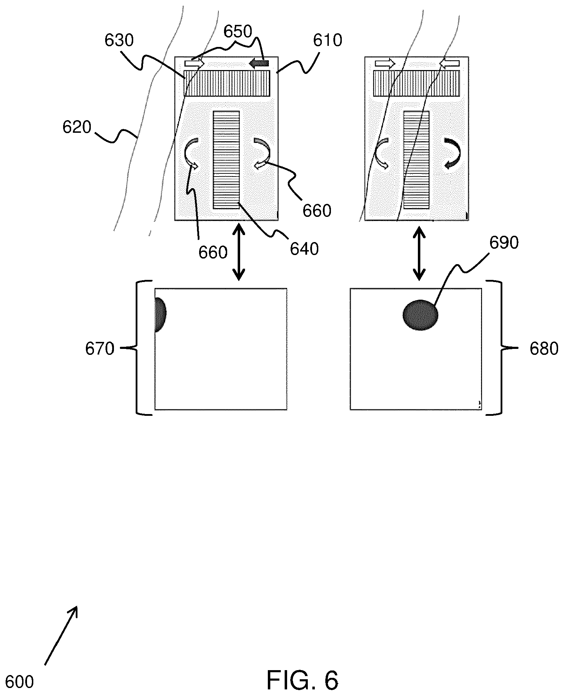

Figure 6 shows a graphical representation 600 of an example of guided sensor placement using a T-shaped array and visual indicators disposed on the sensor.

A sensor 610 is shown placed in close proximity to a vessel of interest 620. The sensor comprises a first transducer array 630, which in this case generates a cross sectional view of the vessel, and a second transducer array 640 arranged orthogonally to the first transducer array. The sensor patch may also include further transducer arrays.

Further, the sensor comprises translation visual indicators 650 and rotational visual indicators 660. In operation, a translation visual indicator may be illuminated to provide a user with a guidance signal to move the patch in the indicated direction. Similarly, a rotational visual indicator may be illuminated to provide a user with a guidance signal to rotate the patch in the indicated direction.

In the example shown in Figure 6, the sensor is placed such that the first transducer array captures an imaging plane that includes an incomplete view 670 of the vessel of interest. The sensor may be initialized by way of a suitable user input, for example a button located on the sensor patch.

Only the first transducer array may be activated at first in order to secure a complete cross sectional view of the vessel of interest. Alternatively, both the first and second transducer arrays may be activated simultaneously.

In this case, the ultrasound sensor patch captures duplex (B-mode and color Doppler) data, which may be streamed to a processor of a connected ultrasound system.

A segmentation algorithm, as described above, is employed to detect the vessels within the imaging plane based on the color Doppler ultrasound data (for example, by searching for the circular appearance of the vessels). The goal in the example shown in Figure 6 is to place the patch on the common carotid artery rather than the interior jugular vein. An indicator communicates a successful completion of this step if the type of the detected vessel is identified as an artery rather than a vein.

In this instance, the initial view of the vessel is incomplete. Accordingly, a translation visual indicator may be activated on the sensor patch, thereby guiding the user to move the patch to a complete view of the vessel 680.

To ensure that the segmented region 690 is indeed the desired vessel, such as an artery and not a vein or a noise artifact, pulse wave Doppler ultrasound data is acquired from the vessel as described above and the features of the flow extracted.

It should be noted that the data need not be visible to the user, but may be performed within the system by the algorithm. The ultrasound sensor patch may be a wearable patch that may be temporarily fixed to a subject, thereby allowing them to move freely while the patch is in operation.

In another example, the patch may include a 2D array of transducer elements capable of performing 3D ultrasound imaging. In this case, an x-plane could be used to help in vessel alignment in an analogous way to the first transducer array described above. An x-plane is suggested, rather than using the full 2D array, as this reduces the number of channels required to align the patch, which reduces the power consumption and data rate, and/or allows for higher frame rates. In this case, the patch may simply be placed on an area of interest without requiring iterative movement or adjustment of the patch. All elements of the array may be activated to find the sub-set of elements that best aligns with the vessel axis. There would be no (or minimal) training or expertise required for patch placement in such an example.

In a further example, the linear transducer arrays of the sensor patch described in Figure 6 may be replaced with 1.5D transducer elements, which would increase the field of view of the imaging plane and may minimize the iterations needed to search for the vessel and/or to align the sensor patch to the vessel.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A computer-implemented method (100) for identifying the type of a vessel of a subject based on Doppler ultrasound data acquired from the subject, the method comprising:

   receiving (110) ultrasound data acquired from the subject;
   generating (120) color Doppler ultrasound data from the received ultrasound data;
   segmenting (130) a representation of a vessel of the subject from the color Doppler ultrasound data;
   obtaining (140) pulse wave Doppler ultrasound data from the segmented vessel representation;
   applying (150) a feature extraction algorithm to the pulse wave Doppler ultrasound data, thereby extracting a feature of the flow within the segmented vessel; and
   identifying (160) the type of the vessel as an artery or a vein based on the extracted feature of the flow.

2. A computer-implemented method (100) as claimed in claim 1, wherein obtaining the pulse wave Doppler ultrasound data from the segmented vessel comprises:

   identifying the center of the vessel based on the color Doppler ultrasound data;
   generating a sampling volume at the center of the vessel; and
   acquiring the pulse wave Doppler ultrasound data from the sampling volume.

3. A computer-implemented method (100) as claimed in any of claim 1 to 2, wherein the feature extraction algorithm comprises:

   extracting (170) a maximum frequency envelope from the pulse wave Doppler ultrasound data;
   normalizing (175) the extracted maximum frequency envelope;
   determining a feature of the normalized maximum frequency envelope; and
   extracting (185) the feature of the normalized maximum frequency envelope as a feature of the flow.

4. A computer-implemented method (100) as claimed in claim 3, wherein determining a feature of the normalized maximum frequency envelope comprises generating (180) a distribution of the normalized maximum frequency envelope with a histogram comprising a plurality of bins.

5. A computer-implemented method (100) as claimed in claim 4, wherein the feature of the normalized maximum frequency envelope comprises one or more of:

   a skewness of the envelope;
   a maximum bin ratio; and
   a bin edge roll-off

6. A computer-implemented method (100) as claimed in any of claims 1 to 5, wherein identifying the type of the vessel as an artery or a vein based on the extracted feature of the flow comprises applying a machine learning algorithm to the extracted feature.

7.  A computer-implemented method (100) as claimed in any of claims 1 to 6, wherein identifying the type of the vessel as an artery or a vein based on the extracted feature of the flow comprises performing a color distribution analysis on the color Doppler ultrasound data.

8.  A computer-implemented method (100) as claimed in claim 7, wherein performing a color distribution analysis on the color Doppler ultrasound data comprises:

    obtaining probe orientation data relating to the orientation of the ultrasound probe during the acquisition of the color Doppler ultrasound data; and
    classifying the vessel as an artery or a vein based on a combination of probe orientation data and color Doppler data.

9.  A computer-implemented method (100) as claimed in any of claims 1 to 8, wherein the method further comprises:

    generating a guidance instruction based on the identification of the vessel; and
    presenting the guidance instruction to the user.

10. A computer program comprising computer program code means which is adapted, when said computer program is run on a computer, to implement the method of any of claims 1 to 9.

11. A processor for identifying the type of a vessel of a subject based on Doppler ultrasound data acquired from a subject, wherein the processor is adapted to:

    obtain ultrasound data from the subject by way of an ultrasound probe;
    generate color Doppler ultrasound data from the received ultrasound data;
    segment a representation of a vessel of the subject from the color Doppler ultrasound data;
    obtain pulse wave Doppler ultrasound data from the segmented vessel representation;
    apply a feature extraction algorithm to the pulse wave Doppler ultrasound data, thereby extracting a feature of the flow within the segmented vessel; and
    identify the type of the vessel as an artery or a vein based on the extracted feature of the flow.

12. A processor as claimed in claim 11, wherein when obtaining the pulse wave Doppler ultrasound data from the segmented vessel, the processor is adapted to:

    identify the center of the vessel based on the color Doppler ultrasound data;
    generate a sampling volume at the center of the vessel; and
    acquire the pulse wave Doppler ultrasound data from the sampling volume.

13. A processor as claimed in any of claims 11 to 12, wherein when applying the feature extraction algorithm, the processor is adapted to:

    extract a maximum frequency envelope from the pulse wave Doppler ultrasound data;
    normalize the extracted maximum frequency envelope;
    determine a feature of the normalized maximum frequency envelope; and
    extract the feature of the normalized maximum frequency envelope as a feature of the flow.

14. An ultrasound imaging system (2) comprising:

    a processor as claimed in any of claims 11 to 13; and
    an ultrasound imaging probe, wherein the ultrasound probe comprises an ultrasound patch (610).

15. An ultrasound imaging system as claimed in claim 14, wherein:

    the system further comprises a display unit, and wherein the processor is further adapted to generate a guidance instruction based on the identification of the type of the vessel and generate a display instruction to cause the display unit to display the guidance instruction; or
    the ultrasound patch (610) further comprises a guidance indicator, and wherein the processor is further adapted to generate a guidance instruction based on the identification of the type of the vessel and control the guidance indicator to provide the guidance instruction to the user.

FIG. 1

```
┌─────────────────────────┐
│                         │        110
│  Receive ultrasound data│ ⌇⌇
│                         │
└─────────────────────────┘
            │
            ▼
┌─────────────────────────┐
│                         │        120
│ Generate color Doppler  │ ⌇⌇
│         data            │
└─────────────────────────┘
            │
            ▼
┌─────────────────────────┐
│                         │        130
│ Segment representation  │ ⌇⌇
│      of a vessel        │
└─────────────────────────┘
            │
            ▼
┌─────────────────────────┐
│                         │        140
│ Obtain pulse wave       │ ⌇⌇
│ Doppler ultrasound data │
└─────────────────────────┘
            │
            ▼
┌─────────────────────────┐
│                         │        150
│ Apply a feature         │ ⌇⌇
│ extraction data         │
└─────────────────────────┘
            │
            ▼
┌─────────────────────────┐
│                         │        160
│ Identify vessel as      │ ⌇⌇
│ artery or vein          │
└─────────────────────────┘
```

100

FIG. 2

FIG. 3

FIG. 4

270

250    260

FIG. 5

FIG. 6

Europäisches
Patentamt

European
Patent Office

Office européen
des brevets

## EUROPEAN SEARCH REPORT

Application Number

EP 20 18 1679

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/115005 A1 (KONINKLIJKE PHILIPS NV [NL]) 11 June 2020 (2020-06-11)<br><br>* abstract *<br>* page 14, line 24 - line 25 *<br>* paragraph bridging page 14 and 15 *<br>* page 15, line 3 - line 8 *<br>* page 15, line 5 - line 18 *<br>* figure 7 *<br>* page 16, line 19 - line 31 *<br>----- | 1,2,8, 10-12, 14,15 | INV.<br>A61B8/00<br>A61B8/08 |
| X | WO 2017/108864 A1 (KONINKLIJKE PHILIPS NV [NL]) 29 June 2017 (2017-06-29)<br>* paragraph [0011] *<br>* paragraph [0043] - paragraph [0045] *<br>* paragraph [0033] *<br>* paragraph [0029] *<br>----- | 1,3-5,7, 10,11,13 | |
| X | WO 2013/088320 A1 (KONINKL PHILIPS ELECTRONICS NV [NL])<br>20 June 2013 (2013-06-20)<br>* page 9, line 32 - page 10, line 26 *<br>* figure 5 *<br>* page 10, line 1 - line 4 *<br>* page 12, line 12 - line 33 *<br>----- | 1,3,4,6, 7,9-11, 13 | |

TECHNICAL FIELDS
SEARCHED      (IPC)

A61B

The present search report has been drawn up for all claims

| Place of search | Date of completion of the search | Examiner |
|---|---|---|
| Munich | 9 November 2020 | Ordavo, Ivan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 3 928 709 A1**

## ANNEX TO THE EUROPEAN SEARCH REPORT
## ON EUROPEAN PATENT APPLICATION NO.

EP 20 18 1679

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2020

| Patent document cited in search report | | Publication date | Patent family member(s) | | Publication date |
|---|---|---|---|---|---|
| WO 2020115005 | A1 | 11-06-2020 | EP | 3669786 A1 | 24-06-2020 |
| | | | WO | 2020115005 A1 | 11-06-2020 |
| WO 2017108864 | A1 | 29-06-2017 | NONE | | |
| WO 2013088320 | A1 | 20-06-2013 | BR | 112014014288 A2 | 13-06-2017 |
| | | | CN | 103997972 A | 20-08-2014 |
| | | | EP | 2790585 A1 | 22-10-2014 |
| | | | JP | 6265909 B2 | 24-01-2018 |
| | | | JP | 2015500122 A | 05-01-2015 |
| | | | RU | 2014129216 A | 10-02-2016 |
| | | | US | 2014343431 A1 | 20-11-2014 |
| | | | WO | 2013088320 A1 | 20-06-2013 |

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin

**Non-patent literature cited in the description**

- **CHAN, T.F. ; SANDBERG Y. B.** Active contours without edges for Vector-valued Image. *Journal of Visual Communication and Image Representation,* 2000, vol. 11, 130-141
- **CHAN, T. F. ; VESE, L. A.** Active contours without edges. *IEEE Transactions on Image Processing,* 2001, vol. 10 (2), 266-277
- **CHAN, T. F. ; VESE, L. A.** A Multiphase level set framework for image segmentation using the Mumford and Shah model. *International Journal of Computer Vision,* 2002, vol. 50 (3), 271-293
- **SOBEL, I. ; FELDMAN, G.** A 3x3 Isotropic Gradient Operator for Image Processing. *Stanford Artificial Intelligence Project (SAIL),* 1968
- **CANNY, J.** A Computational Approach To Edge Detection. *IEEE Trans. Pattern Analysis and Machine Intelligence,* 1986, vol. 8 (6), 679-698